# EUROPÄISCHE PATENTSCHRIFT

(11) **EP 1 827 282 B1**
(45) Veröffentlichungstag und Bekanntmachung des Hinweises auf die Patenterteilung: **19.01.2011**
(21) Anmeldenummer: 05755307.5
(22) Anmeldetag: 29.06.2005
(51) Int. Cl.: A61B 19/02, A61B 19/00, A61F 2/18

(54) **VORRICHTUNG ZUR LÄNGENEINSTELLUNG AN MITTELOHRIMPLANTATEN**
AUXILIARY ELEMENT FOR ADJUSTING THE LENGTH OF MIDDLE-EAR IMPLANTS
ELEMENT AUXILIAIRE POUR LE REGLAGE LONGITUDINAL D'IMPLANTS DE L'OREILLE MOYENNE

(30) Priorität: 12.11.2004 DE 202004017593 U
(43) Veröffentlichungstag der Anmeldung: 05.09.2007
(73) Patentinhaber: Heinz Kurz GmbH Medizintechnik, 72144 Dusslingen (DE)
(72) Erfinder: STEINHARDT, Uwe, 72145 Hirrlingen (DE); KURZ, Heinz, 72144 Dusslingen (DE)
(74) Vertreter: Kohler Schmid Möbus
(86) Internationale Anmeldenummer: PCT/EP2005/006972
(87) Internationale Veröffentlichungsnummer: WO 2006/050761

(56) Entgegenhaltungen:
- EP-A- 1 449 499
- DE-U1- 20 201 303
- GB-A- 2 257 911
- US-A- 4 288 066

## Beschreibung

Die Erfindung betrifft eine Vorrichtung zur Ermittlung der benötigten Länge einer Mittelohrprothese mit einem scheibenförmigen Basisteil, an dem unterschiedlich lange Prothesenattrappen befestigt sind, die mittels eines Applikators nach dem Lösen vom Basisteil zur Längenermittlung während einer Operation in das Mittelohr eines Patienten eingeführt werden können.

Eine derartige Vorrichtung ist bekannt aus der EP 1 449 499 A2.

Die Vorrichtung dient als Hilfsmittel zur Längeneinstellung von Mittelohrimplantaten, insbesondere Teil- oder Vollprothesen, während einer Operation am menschlichen Ohr. Dabei werden ganz oder teilweise fehlende Gehörknöchelchen des menschlichen Mittelohrs, die den Schall vom Trommelfell zum Steigbügel oder zur Steigbügelfußplatte übertragen sollen, durch eine entsprechende individuell an die speziellen Verhältnisse des jeweiligen Patienten anzupassende Mittelohprothese ersetzt. Da die Abmessungen und Geometrien im Ohr jedes Menschen unterschiedlich sind, muss insbesondere der Abstand zwischen Trommelfell und der Steigbügelfußplatte bei einer Totalrekonstruktion oder der Abstand zwischen Trommelfell und Steigbügelköpfchen bei einer Partialrekonstruktion genau ermittelt werden, bevor eine entsprechende Prothese eingesetzt werden kann. Zu diesem Zweck werden Prothesenattrappen in unterschiedlichen Größen für die exakte Längenbestimmung während der Operation in das Mittelohr des Patienten eingeführt.

Da derartige Prothesenattrappen naturgemäß sehr klein sind, gestaltet sich ihre Handhabung, insbesondere ihre Reinigung und Sterilisation, recht schwierig und aufwändig. Daher wurde die in der oben zitierten EP 1 449 499 A2 beschriebene Vorrichtung entwickelt, mit welcher die Prothesenattrappen in leicht zu handhabender Weise am Umfang eines Basisteils mittels Stegen befestigt zur Verfügung gestellt werden. Nach ihrem Lösen vom Basisteil können die Attrappen mittels eines geeigneten Applikators zum Zweck der Längenermittlung in das Mittelohr des Patienten eingeführt werden.

Aufgabe der vorliegenden Erfindung ist es demgegenüber, eine gattungsgemäße Vorrichtung der eingangs beschriebenen Art dahin gehend zu verbessern, dass für den Operateur die Handhabung der Prothesenattrappen während der Operation weiter erleichtert wird, wobei keine zusätzlichen gesonderten Teile eingesetzt werden sollen, sondern die Handhabungsmittel kompakt an der Vorrichtung selbst vorhanden bzw. integriert sind.

Erfindungsgemäß wird diese Aufgabe auf ebenso überraschend einfache wie wirkungsvolle Art und Weise dadurch gelöst, dass am Basisteil Hilfseinrichtungen zur Vermessung und/oder Bearbeitung der einzusetzenden Mittelohrprothese vorgesehen sind. Während bei der bekannten Vorrichtung lediglich die Attrappen selbst am Basisteil angebracht waren, kann nunmehr der Operateur das scheibenförmige Basisteil für eine Vermessung und Bearbeitung der einzusetzenden Mittelohrprothese verwenden, ohne dafür weitere Teile und zusätzlichen Platz zu benötigen. Da die erfindungsgemäße Vorrichtung ohnehin steril angeliefert werden muss, sind auch die Vermessungs- und Bearbeitungseinrichtungen am Basisteil automatisch steril, sodass die einzusetzende bzw. anzupassende Mittelohrprothese darauf während der Operation problemlos abgelegt und verarbeitet werden kann.

Die Hilfseinrichtungen umfassen eine oder mehrere, auf der Ober- und/oder Unterseite des scheibenförmigen Basisteils vorgesehene Ausnehmungen, die einen lichten Durchmesser aufweisen, welcher dem Außendurchmesser einer Kopfplatte der einzusetzenden Mittelohrprothese entspricht. In diesen Ausnehmungen des Basisteils kann dann die Kopfplatte der gewünschten Mittelohrprothese während der Operation eingelegt und bearbeitet werden.

Eine vorteilhafte Weiterbildung dieser Ausführungsform sieht vor, dass unter jeder Ausnehmung eine in das Basisteil hineinragende Kavität vorgesehen ist, die insbesondere der Aufnahme einer unter der Kopfplatte der Mittelohrprothese angebrachten Glocke zur Befestigung der Mittelohrprothese auf dem Steigbügel des Mittelohres oder der Aufnahme eines unter der Kopfplatte der Mittelohrprothese angebrachten Stempels zur Auflage der Mittelohrprothese auf der Steigbügelfußplatte des Mittelohrs dient. Diese geometrische Gestaltung erleichtert die Handhabung der Prothese erheblich und verhindert ein Herausrutschen derselben aus der Ausnehmung im Basisteil, da die entsprechende Glocke oder der Stempel in die darunter liegende Kavität ragt.

Eine für die Handhabung besonders günstige Formgestaltung besteht darin, dass die Kontur des lichten Durchmessers der Ausnehmungen abschnittsweise der Außenkontur der Kopfplatte der Mittelohrprothese entspricht, aber in mindestens einem Abschnitt radial gegenüber dem maximalen Außendurchmesser der Kopfplatte nach außen hin erweitert ist. Auf diese Weise ist ein Verklemmen der in die Ausnehmung des Basisteils eingelegten Kopfplatte der Mittelohrprothese unschädlich, da diese über die Erweiterung des maximalen Außendurchmessers der Ausnehmung leicht wieder gelöst werden kann.

Ganz besonders bevorzugt ist eine Weiterbildung der oben beschriebenen Ausführungsform der erfindungsgemäßen Vorrichtung, bei welcher im Basisteil mehrere, vorzugsweise über den Umfang des Basisteils verteilt angeordnete Ausnehmungen vorgesehen sind, die den Außendurchmessern der Kopfplatten von Mittelohrprothesen entsprechen und jeweils unterschiedliche Tiefen aufweisen. Dadurch können in unterschiedlichen Ausnehmungen des Basisteils unterschiedlich lange Mittelohrprothesen aufgenommen worden, sodass für die jeweils benötigte optimale Länge genau die Richtige Ausnehmung auf dem Basisteil vorgehalten werden kann.

Die Handhabung der erfindungsgemäßen Vorrichtung für den Operateur lässt sich noch weiter erleichtern, wenn auf dem Basisteil neben jeder Ausnehmung eine Markierung oder Beschriftung vorgesehen ist, die mit der jeweiligen Tiefe der entsprechenden Ausnehmung bzw. der Länge der darin aufnehmbaren Kopfplatte und/oder mit der Größe des lichten Durchmessers der entsprechenden Ausnehmung bzw. dem Außendurchmesser der Kopfplatte und/oder mit Form der Ausnehmung bzw. der Kopfplatte korreliert. So kann der Operateur mit einem Blick die richtige Ausnehmung für die gewünschte Größe der einzusetzenden Mittelohrprothese auswählen.

Eine weitere bevorzugte Ausführungsform der erfindungsgemäßen Vorrichtung zeichnet sich dadurch aus, dass die Hilfseinrichtungen mindestens eine konusförmige Erhebung mit vorzugsweise abgerundeter Spitze umfassen, die von einer Seite des Basisteils wegragt und zur Ausweitung des lichten Durchmessers einer unter der Kopfplatte der Mittelohrprothese angebrachten Glocke zur Befestigung der Mittelohrprothese auf dem Steigbügel des Mittelohres ausgebildet ist. Damit kann die Glocke der Partialprothese, falls sie nicht genau auf den Steigbügel des Mittelohrs passen sollte, genau auf die tatsächlichen geometrischen Verhältnisse im Ohr des Patienten angepasst werden, ohne dass dazu der Operateur eine weitere sterile Vorrichtung in Benutzung nehmen müsste.

Vorteilhaft ist in diesem Zusammenhang eine Weiterbildung aus einer Kombination der oben beschriebenen Ausführungsformen, bei der die konusförmige Erhebung auf derselben Seite des scheibenförmigen Basisteils angeordnet ist wie die Ausnehmung(en) zur Aufnahme der Kopfplatte einer Mittelohrprothese. Dies erleichtert die Handhabung erheblich, weil die in den Ausnehmungen vorbearbeitete Prothese auf der gleichen Seite des scheibenförmigen Basisteils weiterverarbeitet werden kann, ohne dass das Basisteil umgedreht werden muss. Insbesondere kann die konusförmige Erhebung in einer Kavität auf dieser Seite des scheibenförmigen Basisteils nahe den Ausnehmungen zur Aufnahme der Kopfplatte angeordnet sein.

Eine weitere besonders bevorzugte Ausführungsform der erfindungsgemäßen Vorrichtung zeichnet sich dadurch aus, dass die Hilfseinrichtungen mindestens eine, vorzugsweise mehrere Vertiefungen im Basisteil umfassen, die zur Aufnahme und gegebenenfalls zur Vermessung und/oder Bearbeitung eines Knorpel-oder Faszienscheibchens ausgebildet sind, welches als Zwischeneinlage zwischen der Mittelohrprothese und dem Trommelfell zum mechanischen Schutz des Letzteren dient. Derartige Vertiefungen zur Bearbeitung von Knorpel- oder Faszienscheibchen sind an sich unter der Bezeichnung "Templates" bekannt, jedoch üblicherweise in eigens dafür vorgesehenen weiteren Vorrichtungen vorgehalten, die für die Operation wiederum steril gemacht werden müssen und zusätzlichen Raum einnehmen würden, während mit der erfindungsgemäßen Vorrichtung auch diese Bearbeitung kompakt auf dem scheibenförmigen Basisteil erfolgen kann.

Vorzugsweise weisen die Vertiefungen zur Aufnahme von Knorpel- oder Faszienscheibchen einen runde oder ovale Kontur auf. Dies entspricht der üblichen Formgebung derartiger Scheibchen bei einschlägigen Operationen. Da die geometrischen Verhältnisse, wie bereits oben mehrfach erwähnt, bei jedem Patienten anders sind, ist es von Vorteil, wenn bei der erfindungsgemäßen Vorrichtung im Basisteil mehrere Vertiefungen mit unterschiedlich großen lichten Durchmessern und/oder unterschiedlichen Tiefen vorgesehen sind, sodass die im Einzelfall erforderlichen Knorpel- oder Faszienscheibchen mit der vorhandenen Vorrichtung ohne den Einsatz von zusätzlichen weiteren Vorrichtungen präpariert werden können.

Bevorzugt ist auch eine Ausführungsform der erfindungsgemäßen Vorrichtung, bei der die Hilfseinrichtungen mindestens ein im Wesentlichen ebenes Präparationsfeld auf zumindest einer Seite des scheibenförmigen Basisteils umfassen. Auf diesem Präparationsfeld können weitere Verarbeitungsschritte an der einzusetzenden Mittelohrprothese und/oder an etwa vorbereiteten Knorpel- oder Faszienscheibchen vorgenommen werden. Dabei ist es für den Operateur äußerst hilfreich, wenn bei Weiterbildungen dieser Ausführungsform zumindest an einer Stelle des Präparationsfeldes eine Längenskala in der Oberfläche des Basisteils angebracht ist, die vorzugsweise in Millimetern geeicht ist. Damit lassen sich auf einen Blick und ohne weitere Hilfsmittel absolute Größenvergleiche oder Vermessungen vornehmen.

Besonders bevorzugt ist eine Kombination der oben beschriebenen Ausführungsformen, bei welcher die Vertiefungen zur Aufnahme von Knorpel- oder Faszienscheibchen im Präparationsfeld angeordnet sind. Dadurch wird ein "nahtloser" Übergang der einzelnen Präparationsschritte im selben engen Raumbereich ermöglicht.

Eine vorteilhafte und besonders einfache Handhabung ermöglicht auch eine Weiterbildung der Erfindung, bei welcher die Ausnehmungen zur Aufnahme der Kopfplatte einer Mittelohrprothese einerseits und das Präparationsfeld sowie gegebenenfalls eine oder mehrere Vertiefungen zur Aufnahme von Knorpel- oder Faszienscheibchen andererseits auf zwei unterschiedlichen, vorzugsweise gegenüberliegenden Seiten des scheibenförmigen Basisteils angeordnet sind.

Ganz besonders bevorzugt ist eine Ausführungsform der erfindungsgemäßen Vorrichtung, wie sie an sich bereits aus der eingangs zitierten EP 1 449 499 A2 bekannt ist, bei der die Prothesenattrappen an Stegen satellitenartig über den Außenumfang des scheibenförmigen Basisteils verteilt befestigt sind. Die Befestigung erfolgt in der Regel über leicht abbrechbare Kunststoffstege, was es dem Operateur ermöglicht, problemlos eine Prothesenattrappe der gewünschten Größe vom Basisteil zu entfernen und zu Vermessungszwecken im Mittelohr des Patienten einzusetzen.

Um beim Transport die relativ kleinen und zarten Prothesenattrappen gegen mechanische Beschädigungen zu schützen, sind bei einer Weiterbildung dieser Ausführungsform über den Außenumfang des scheibenförmigen Basisteils verteilt jeweils zwischen zwei benachbarten Prothesenattrappen insbesondere stabförmige Schutzvorsprünge vorgesehen, die die Prothesenattrappen in radialer Richtung überragen.

Als besonders vorteilhaft hat sich dabei eine geometrische Ausgestaltung der erfindungsgemäßen Vorrichtung herausgestellt, die sich dadurch auszeichnet, dass das scheibenförmige Basisteil einen polygonartigen, insbesondere einen sechseckigen oder achteckigen Außenumfang aufweist und dass die Schutzvorsprünge in radialer Richtung an den Ecken des Basisteils nach außen wegragen.

Um dem Operateur unnötige Handgriffe zu ersparen, sollten bei der erfindungsgemäßen Vorrichtung die am Basisteil befestigten Prothesenattrappen unterschiedliche Größen, insbesondere unterschiedliche Längen aufweisen. Die Arbeit des Operateurs kann auch durch Weiterbildungen der oben genannten Ausführungsform der Erfindung erleichtert werden, bei denen die Prothesenattrappen am Außenumfang des Basisteils jeweils in der radialen Nachbarschaft einer Ausnehmung zur Aufnahme der Kopfplatte einer Mittelohrprothese angeordnet sind und bei denen die Größe der Ausnehmung der Größe der ihr jeweils benachbarten Prothesenattrappe entspricht. Auf diese Weise ist eine schnelle optische Zuordnung möglich, ohne dass erst Vermessungen vorgenommen werden müssten.

Besonders preiswert und in großen Stückzahlen lässt sich die erfindungsgemäße Vorrichtung herstellen, wenn dass das Basisteil samt Stegen und Prothesenattrappen im Spritzgussverfahren aus Kunststoff gefertigt sind, vorzugsweise einstückig.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1: eine Ausführungsform der erfindungsgemäßen Vorrichtung mit Prothesenattrappen für Mittelohr-Totalprothesen in einer schematischen Darstellung von schräg oben mit Blickrichtung auf die Seite des Basisteils, in denen die Ausnehmungen und Kavitäten zur Aufnahme der Kopfplatte der Mittelohrprothesen vorgesehen sind;
- Fig. 2: eine Ansicht wie Fig. 1, aber mit einer Ausführungsform mit Prothesenattrappen für Mittelohr-Partialprothesen und Konus zur Verarbeitung der Glocke;
- Fig. 3: eine Ausführungsform wie Fig. 2, aber mit zusätzlichen Schutz- vorsprüngen an den Ecken des scheibenförmigen Basisteils;
- Fig. 4: die Ausführungsform nach Fig. 2 von der Rückseite des Basisteils mit Blick auf die Präparationsfläche für Knorpel- oder Faszienscheibchen;
- Fig. 5a: eine schematische Detailansicht einer Ausnehmung zur Aufnahme der Kopfplatte einer Mittelohrprothese;
- Fig. 5b: die Bearbeitung einer in die Ausnehmung eingelegten Mittelohrprothese mittels Schließzängchen;
- Fig. 5c: die Bearbeitung einer in die Ausnehmung eingelegten Mittelohrprothese mittels Schneidezängchen;
- Fig. 5d: eine nach Fig. 5c bearbeitete Mittelohrprothese nach dem Abschneiden des überstehenden Schaftes;
- Fig. 6a: ein Detail einer Ausführungsform mit konusförmiger Erhebung zur Bearbeitung einer Glocke einer Mittelohr-Partialprothese;
- Fig. 6b: ein größeres Detail aus Fig. 6a mit auf die Konusspitze aufgesetzter Glocke der Mittelohr-Partialprothese;
- Fig. 7: eine vergrößerte Darstellung der Präparationsfläche einer Ausführungsform wie in Fig. 4.

Die erfindungsgemäße Vorrichtung zur Ermittlung der benötigten Länge einer Mittelohrprothese umfasst ein scheibenförmiges Basisteil 10, 20, 30, wie es in sämtlichen Figuren der Zeichnung zu erkennen ist. Dieses wird in der Regel als Kunststoff-Spritzgussteil ausgeführt sein, an dessen Umfang an Stegen angebrachte und leicht entfernbare Prothesenattrappen 11, 11', 11"; 21, 21', 21"; 31, 31', 31" befestigt sind. Diese Prothesenattrappen können mittels eines Applikators nach dem Lösen vom Basisteil 10, 20, 30 zur Längenermittlung während einer Operation in das Mittelohr eines Patienten eingeführt werden.

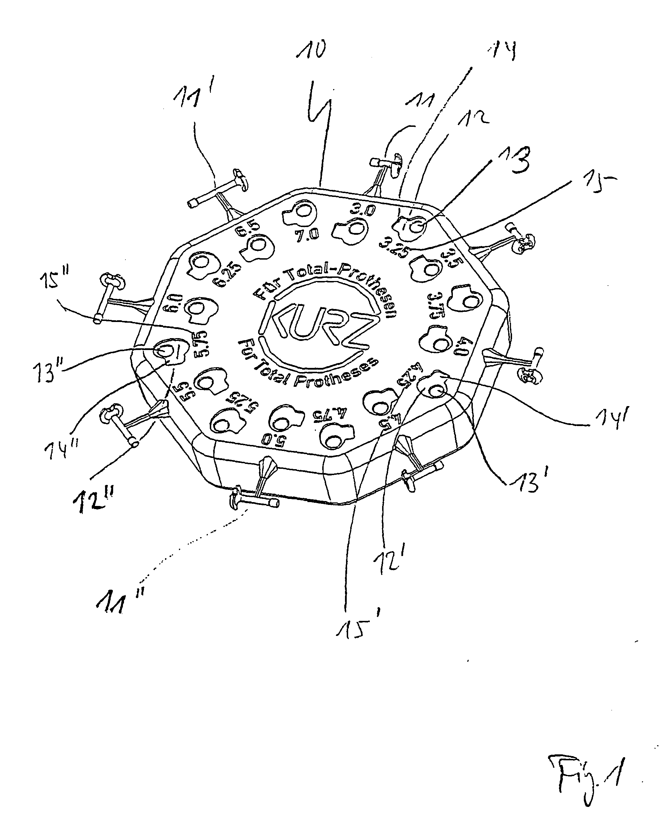

Erfindungsgemäß sind am Basisteil 10, 20, 30 Hilfseinrichtungen zur Vermessung und/oder Bearbeitung der einzusetzenden Mittelohrprothesen vorgesehen. Bei der Ausführungsform nach Fig. 1 umfassen diese Hilfseinrichtungen mehrere auf der Oberseite des scheibenförmigen Basisteils 10 vorgesehene Ausnehmungen 12, 12', 12", die einen lichten Durchmesser aufweisen, welcher dem Außendurchmesser einer Kopfplatte der einzusetzenden Mittelohrprothese entspricht. Unter jeder dieser Ausnehmungen 12, 12', 12" ist eine in das Basisteil 10 hineinragende Kavität 13, 13', 13" vorgesehen, die im Falle der Ausführungsform nach Fig. 1 der Aufnahme eines unter der Kopfplatte der Mittelohrprothese angebrachten Stempels zur Auflage der Mittelohrprothese auf der Steigbügelfußplatte des Mittelohrs dient. Die Kontur des lichten Durchmessers der Ausnehmungen 12, 12', 12" ist jeweils in einem Abschnitt 14, 14', 14" radial gegenüber dem maximalen Außendurchmesser der Kopfplatte nach außen hin erweitert, sodass diese sich selbst bei einer Verklemmung leicht wieder entfernen lässt. Neben jeder Ausnehmung 12, 12', 12" ist eine Beschriftung 15, 15', 15" vorgesehen, die im vorliegenden Ausführungsbeispiel den Operateur über die Tiefe der zugehörigen Kavität 13, 13'. 13" informiert.

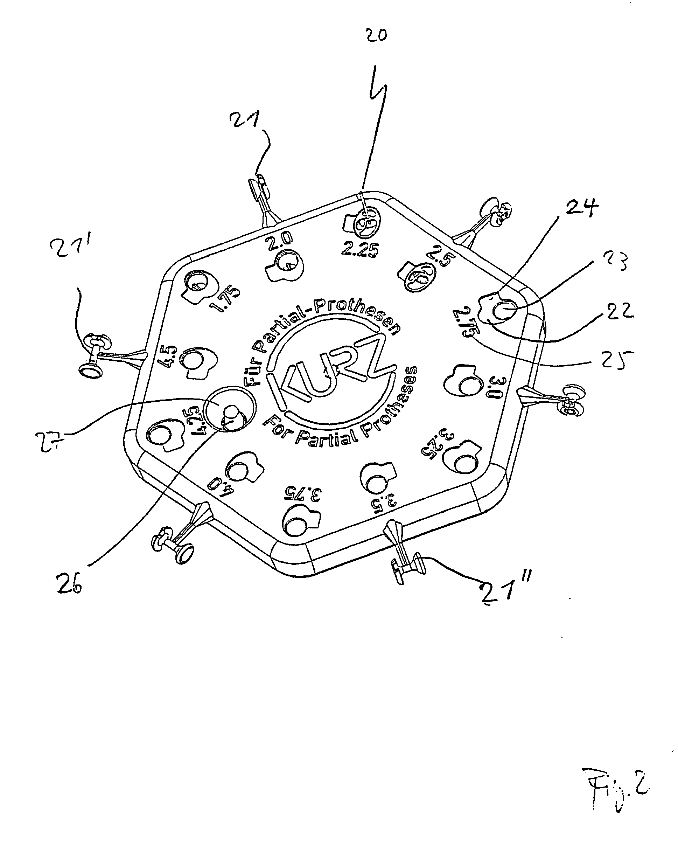

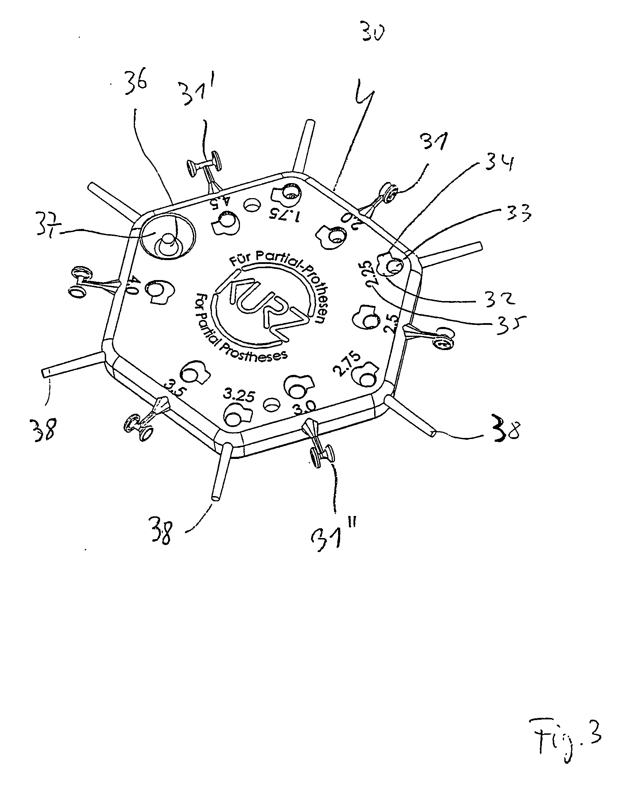

Ähnliche Ausnehmungen 22; 32 mit zugehörigen Kavitäten 23; 33 und radial nach außen erweiterten Abschnitten 24; 34 sowie Beschriftungen 25; 35 sind auch in den Ausführungsformen nach den Figuren 2 und 3 vorgesehen, welche zur Verarbeitung von Mittelohr-Partialprothesen geeignet sind. Die entsprechenden Prothesenattrappen 21, 21', 21"; 31, 31', 31" sind daher entsprechend kürzer als die in Fig. 1 gezeigten Prothesenattrappen 11, 11', 11".

Die Ausführungsformen nach den Fig. 2 und 3 weisen zur Bearbeitung von üblicherweise an Mittelohr-Partialprothesen vorgesehenen Glocken, insbesondere zu deren Aufweitung nach individuellen Gegebenheiten, konusförmige Erhebungen 26; 36 mit abgerundeten Spitzen auf, welche jeweils in einer Vertiefung 27; 37 auf der gleichen Seiten des scheibenförmigen Basisteils 20; 30 vorgesehen sind wie die oben beschriebenen Ausnehmungen 22; 32.

Zusätzlich zu der Ausführungsform nach Fig. 2 weist die Vorrichtung nach Fig. 3 an den Ecken des Basisteils 30 jeweils nach außen radial die Prothesenattrappen 31, 31', 31" überragende Schutzvorsprünge 38 auf, die dem mechanischen Schutz der Prothesenattrappen 31, 31', 31" während des Transports der Vorrichtung dienen.

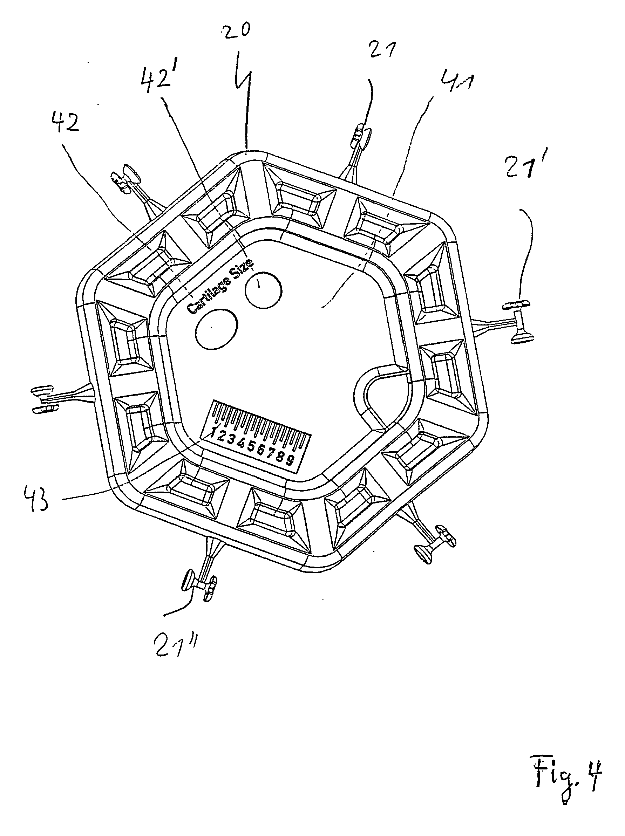

In Fig. 4 ist die Vorrichtung nach Fig. 2 von der Unterseite dargestellt. Man erkennt hier ein im Wesentlichen ebenes Präparationsfeld 41, an dessen Rand unterschiedlich große ovale bzw. runde Vertiefungen 42, 42' zur Aufnahme von Knorpel- oder Faszienscheibchen angeordnet sind, welche als Zwischeneinlage zwischen der Mittelohrprothese und dem Trommelfell zu dessen mechanischem Schutz dienen sollen. Außerdem ist am Rand des Präparationsfeldes eine Längenskala 43 vorgesehen, welche beispielsweise in Millimetern geeicht sein kann und dem Operateur auf einen Blick die absoluten Größen der zu verarbeitenden Teile oder Transplantate anzeigt.

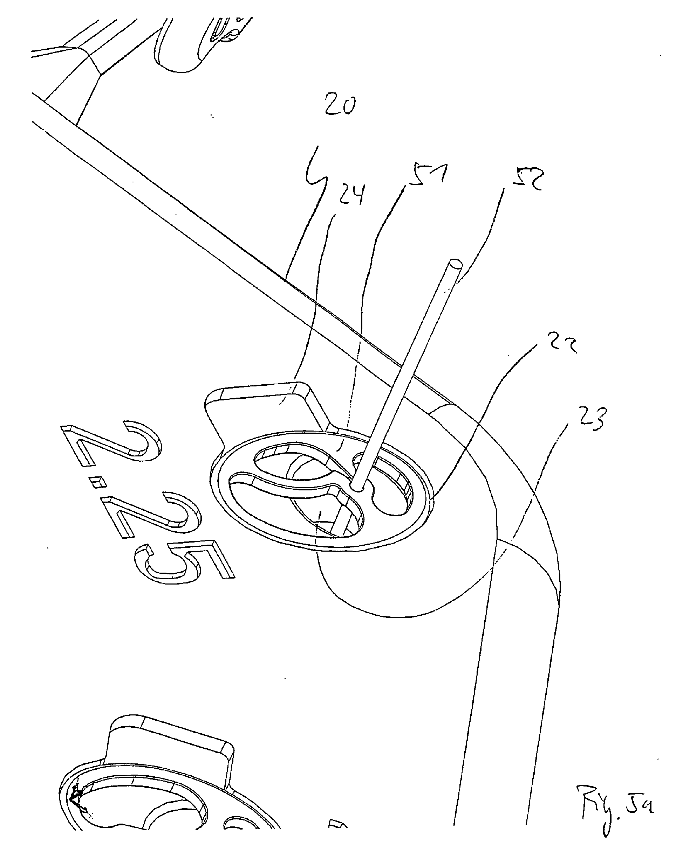

In den Fig. 5a bis 5d ist die Bearbeitung von Mittelohrprothesen in einer der Ausnehmungen 22 eines Basisteils 20 (wie in Fig. 2 dargestellt) gezeigt. In Fig. 5a erkennt man eine Kopfplatte 51 mit einem Schaft 52, die in eine geometrisch passende Ausnehmung 22 eingelegt ist. Die andere Seite des Schafts 52 ragt in die unter der Ausnehmung 22 vorgesehene Kavität 23 des Basisteils 20. Zur Entfernung der Mittelohrprothese aus der Ausnehmung 22 kann die Kopfplatte 51 leicht über den radial ausbauchenden Abschnitt 24 erfasst und herausgedrückt werden.

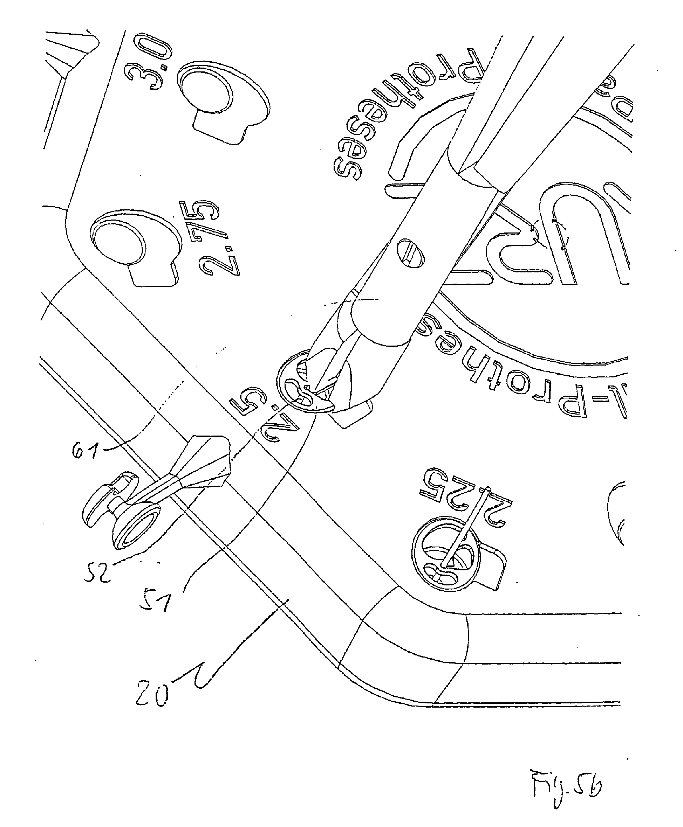

Der Abschnitt 24 dient als Platzerweiterung beim Schließen der Kopfplatte, wie in Fig. 5b gezeigt. Das Herausnehmen der Prothese wird mittels Pinzette am Schaft getätigt, oder man dreht das Kunststoffteil.

In Fig. 5b wird mittels eine Schließzängchens 61 ein Steg der Kopfplatte 51 geradegebogen, damit sich der Abstand schließt und die Kopfplatte kraftschlüssig auf dem Schaft 52 der Prothese fixiert wird.

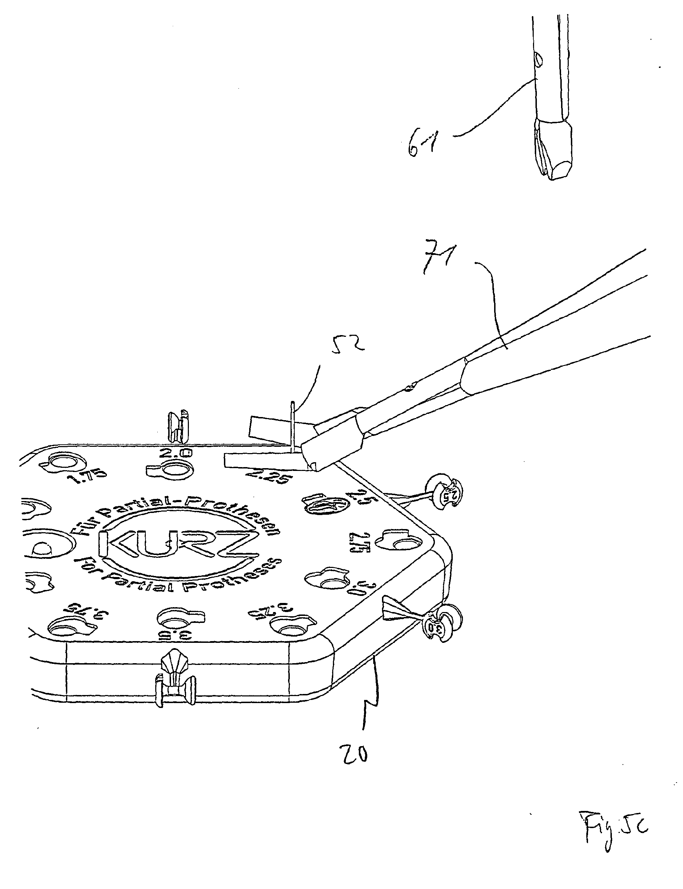

Fig. 5c stellt einen weiteren Arbeitschritt bei der Bearbeitung einer Mittelohr-Partialprothese dar: Hierbei wird mittels eines Schneidezängchens 71 der nach oben überstehende Teil des Schafts 52 abgezwickt.

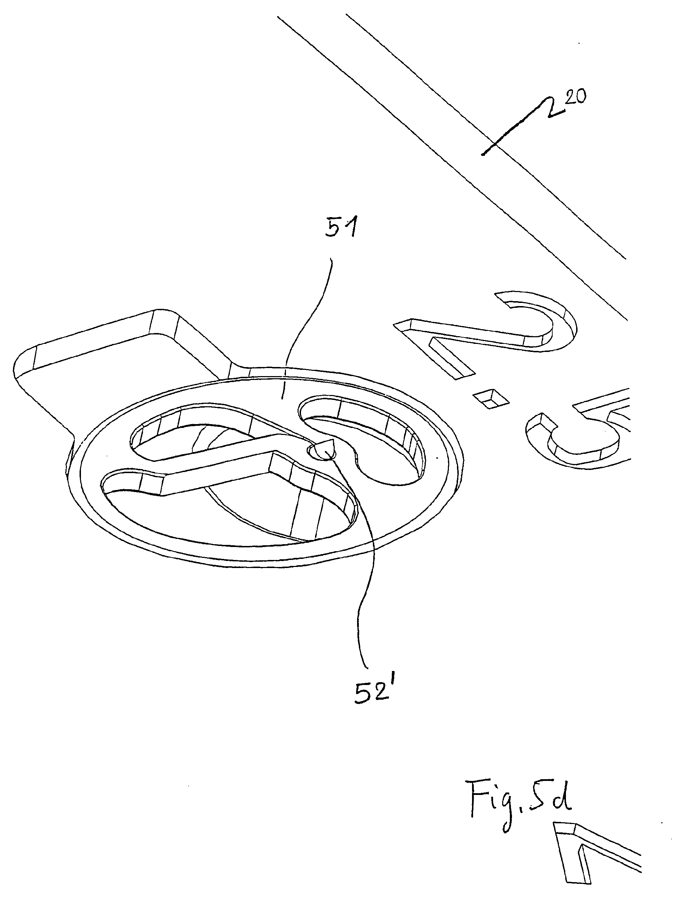

Fig. 5d schließlich zeigt die Situation nach diesem Bearbeitungsschritt, wobei noch ein kleiner spitzer Rest 52' des Schafts 52 erkennbar ist, welcher als Fixierungspin für die später darauf aufliegende Knorpel- oder Faszienplatte dienen wird.

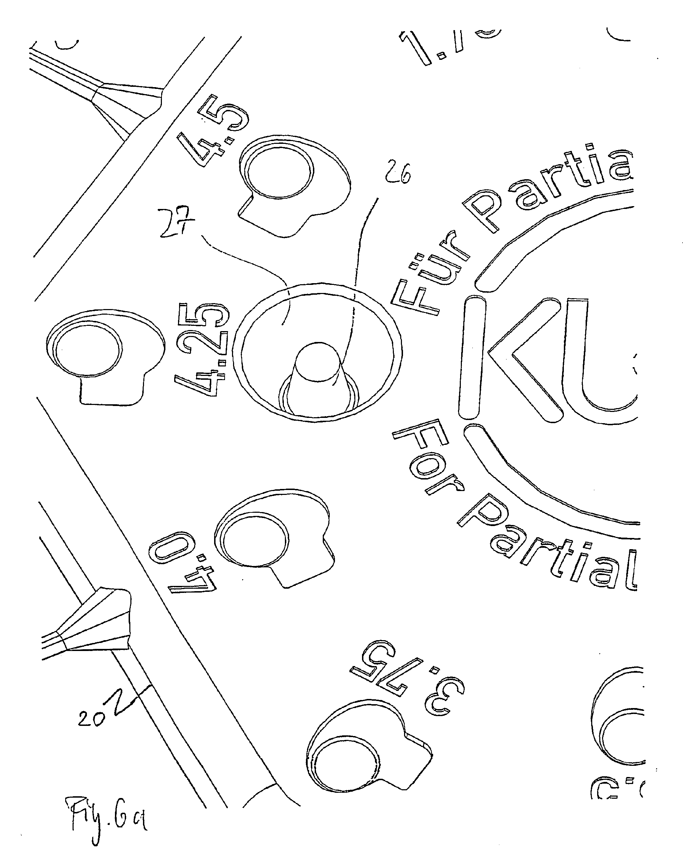

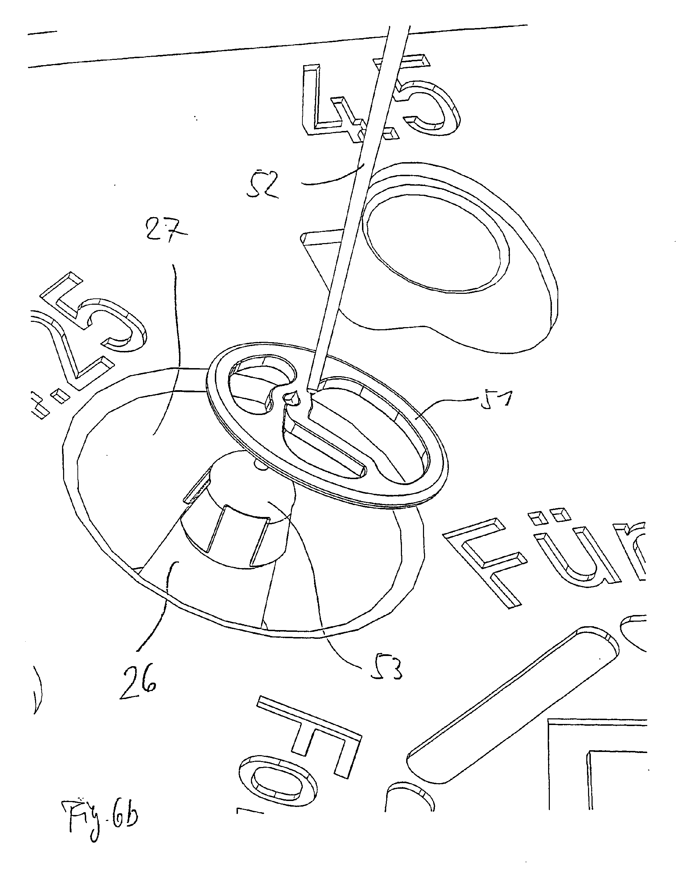

In Fig. 6a ist die konusförmige Erhebung aus der Ausführungsform nach Fig. 2 vergrößert dargestellt. Fig. 6b zeigt in einer noch größeren Vergrößerung den Bearbeitungsschritt des Ausweitens des lichten Durchmessers einer unterhalb der Kopfplatte 51 vorgesehenen Glocke 53 einer Mittelohr-Partialprothese. Die Glocke 53 dient später zur Befestigung der Mittelohrprothese auf dem Steigbügel des Mittelohrs.

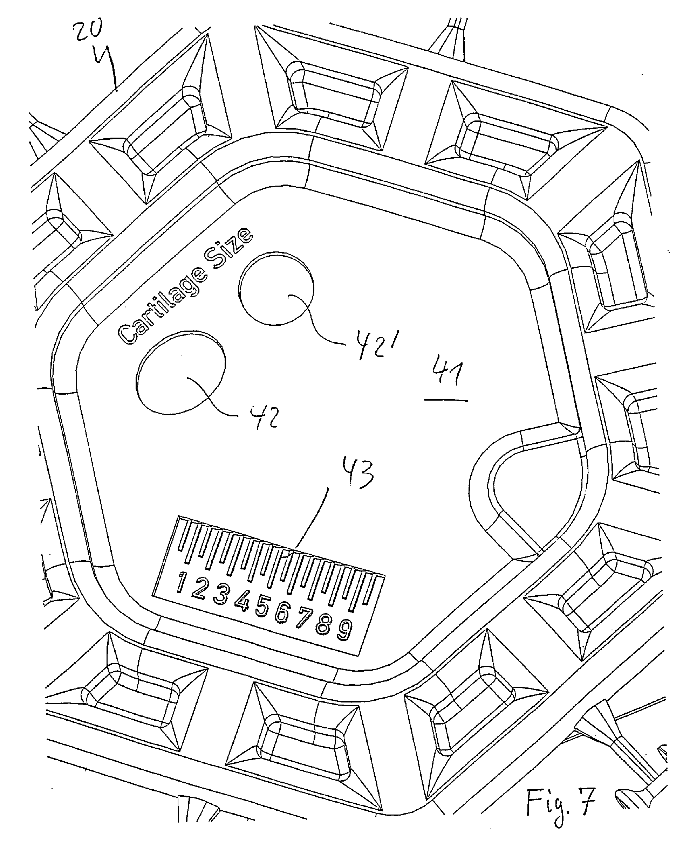

In Fig. 7 schließlich ist nochmals das ebene Präparationsfeld 41 mit den Vertiefungen 42, 42' zur Aufnahme von Knorpel- oder Faszienscheibchen sowie mit einer geeichten Längenskala 43 dargestellt.

## Patentansprüche

1. Vorrichtung zur Ermittlung der benötigten Länge einer Mittelohrprothese, mit einem scheibenförmigen Basisteil (10; 20; 30), an dem unterschiedlich lange Prothesenattrappen (11,11',11"; 21,21',21"; 31,31',31") befestigt sind, die mittels eines Applikators nach dem Lösen vom Basisteil (10; 20; 30) zur Längenermittlung während einer Operation in das Mittelohr eines Patienten eingeführt werden können, **dadurch gekennzeichnet, dass** am Basisteil (10; 20; 30) Hilfseinrichtungen zur Vermessung und/oder Bearbeitung der einzusetzenden Mittelohrprothese vorgesehen sind, und dass die Hilfseinrichtungen eine oder mehrere, auf der Ober- und/oder Unterseite des scheibenförmigen Basisteils (10; 20; 30) vorgesehene Ausnehmungen (12, 12', 12"; 22; 32) umfassen, die einen lichten Durchmesser aufweisen, welcher dem Außendurchmesser einer Kopfplatte (51) der einzusetzenden Mittelohrprothese entspricht.

2. Vorrichtung nach Anspruch 1, **dadurch gekennzeichnet, dass** unter jeder Ausnehmung (12, 12', 12"; 22; 32) eine in das Basisteil (10; 20; 30) hineinragende Kavität (13, 13', 13"; 23; 33) vorgesehen ist, die insbesondere der Aufnahme einer unter der Kopfplatte (51) der Mittelohrprothese angebrachten Glocke (53) zur Befestigung der Mittelohrprothese auf dem Steigbügel des Mittelohres oder der Aufnahme eines unter der Kopfplatte (51) der Mittelohrprothese angebrachten Stempels zur Auflage der Mittelohrprothese auf der Steigbügelfußplatte des Mittelohres dient.

3. Vorrichtung nach einem der Ansprüche 1 oder 2, **dadurch gekennzeichnet, dass** die Kontur des lichten Durchmessers der Ausnehmungen (12, 12', 12"; 22; 32) abschnittsweise der Außenkontur der Kopfplatte (51) der Mittelohrprothese entspricht, aber in mindestens einem Abschnitt (14, 14', 14"; 24; 34) radial gegenüber dem maximalen Außendurchmesser der Kopfplatte (51) nach außen hin erweitert.

4. Vorrichtung nach einem der Ansprüche 1 bis 3, **dadurch gekennzeichnet, dass** im Basisteil (10; 20; 30) mehrere, vorzugsweise über den Umfang des Basisteils (10; 20; 30) verteilt angeordnete Ausnehmungen (12, 12', 12"; 22; 32) vorgesehen sind, die den Außendurchmessern der Kopfplatten (51) von Mittelohrprothesen entsprechen und jeweils unterschiedliche Tiefen aufweisen.

5. Vorrichtung nach Anspruch 4, **dadurch gekennzeichnet, dass** auf dem Basisteil (10; 20; 30) neben jeder Ausnehmung (12, 12', 12"; 22; 32) eine Markierung oder Beschriftung (15, 15', 15"; 25; 35) vorgesehen ist, die mit der jeweiligen Tiefe der entsprechenden Ausnehmung (12, 12', 12"; 22; 32) bzw. der Länge der darin aufnehmbaren Kopfplatte (51) und/oder mit der Größe des lichten Durchmessers der entsprechenden Ausnehmung (12, 12', 12"; 22; 32) bzw. dem Außendurchmesser der Kopfplatte (51) und/oder mit Form der Ausnehmung (12, 12', 12"; 22; 32) bzw. der Kopfplatte (51) korreliert ist.

6. Vorrichtung nach einem der vorhergehenden Ansprüche, **dadurch gekennzeichnet, dass** die Hilfseinrichtungen mindestens eine konusförmige Erhebung (26; 36) mit vorzugsweise abgerundeter Spitze umfassen, die von einer Seite des Basisteils (20; 30) wegragt, vorzugsweise in einer Vertiefung (27; 37) sitzt und zur Ausweitung des lichten Durchmessers einer unter der Kopfplatte (51) der Mittelohrprothese angebrachten Glocke (53) zur Befestigung der Mittelohrprothese auf dem Steigbügel des Mittelohres ausgebildet ist.

7. Vorrichtung nach Anspruch 6, **dadurch gekennzeichnet, dass** die konusförmige Erhebung (26; 36) auf derselben Seite des scheibenförmigen Basisteils (20; 30) angeordnet ist wie die Ausnehmung(en) (22; 32) zur Aufnahme der Kopfplatte (51) einer Mittelohrprothese.

8. Vorrichtung nach einem der vorhergehenden Ansprüche, **dadurch gekennzeichnet, dass** die Hilfseinrichtungen mindestens eine, vorzugsweise mehrere Vertiefungen (42, 42') im Basisteil (10; 20; 30) umfassen, die zur Aufnahme und gegebenenfalls zur Vermessung und/oder Bearbeitung eines Knorpel- oder Faszienscheibchens ausgebildet sind, welches als Zwischeneinlage zwischen der Mittelohrprothese und dem Trommelfell zum mechanischen Schutz des letzteren dient.

9. Vorrichtung nach Anspruch 8, **dadurch gekennzeichnet, dass** die Vertiefungen (42, 42') zur Aufnahme von Knorpel- oder Faszienscheibchen einen runde oder ovale Kontur aufweisen.

10. Vorrichtung nach einem der Ansprüche 8 oder 9, **dadurch gekennzeichnet, dass** im Basisteil (10; 20; 30) mehrere Vertiefungen (42, 42') mit unterschiedlich großen lichten Durchmessern und/oder unterschiedlichen Tiefen vorgesehen sind.

11. Vorrichtung nach einem der vorhergehenden Ansprüche, **dadurch gekennzeichnet, dass** die Hilfseinrichtungen mindestens ein i.w. ebenes Präparationsfeld (41) auf zumindest einer Seite des scheibenförmigen Basisteils (10; 20; 30) umfassen.

12. Vorrichtung nach Anspruch 11, **dadurch gekennzeichnet, dass** zumindest an einer Stelle des Präparationsfeldes (41) eine Längenskala (43) in der Oberfläche des Basisteils (10; 20; 30) angebracht ist, die vorzugsweise in Millimetern geeicht ist.

13. Vorrichtung nach einem der Ansprüche 8 bis 10 sowie einem der Ansprüche 11 oder 12, **dadurch gekennzeichnet, dass** die Vertiefungen (42, 42') zur Aufnahme von Knorpel- oder Faszienscheibchen im Präparationsfeld (41) angeordnet sind.

14. Vorrichtung nach einem der Ansprüche 1 bis 7 sowie einem der Ansprüche 8 bis 13, **dadurch gekennzeichnet, dass** die Ausnehmungen (12, 12', 12"; 22; 32) zur Aufnahme der Kopfplatte (51) einer Mittelohrprothese einerseits und das Präparationsfeld (41) sowie gegebenenfalls eine oder mehrere Vertiefungen (42, 42') zur Aufnahme von Knorpel- oder Faszienscheibchen andererseits auf zwei unterschiedlichen, vorzugsweise gegenüberliegenden Seiten des scheibenförmigen Basisteils (10; 20; 30) angeordnet sind.

15. Vorrichtung nach einem der vorhergehenden Ansprüche, **dadurch gekennzeichnet, dass** die Prothesenattrappen (11,11',11"; 21,21',21"; 31,31',31") an Stegen satellitenartig über den Außenumfang des scheibenförmigen Basisteils (10; 20; 30) verteilt befestigt sind.

16. Vorrichtung nach Anspruch 15, **dadurch gekennzeichnet, dass** über den Außenumfang des scheibenförmigen Basisteils (30) verteilt jeweils zwischen zwei benachbarten Prothesenattrappen (31, 31', 31 ") insbesondere stabförmige Schutzvorsprünge (38) vorgesehen sind, die die Prothesenattrappen (31, 31', 31 ") in radialer Richtung überragen.

17. Vorrichtung nach den Ansprüchen 15 und 16, **dadurch gekennzeichnet, dass** das scheibenförmige Basisteil (30) einen polygonartigen, insbesondere einen sechseckigen oder achteckigen Außenumfang aufweist, und dass die Schutzvorsprünge (38) in radialer Richtung von den Ecken des Basisteils (30) nach außen wegragen.

18. Vorrichtung nach einem der vorhergehenden Ansprüche, **dadurch gekennzeichnet, dass** die Prothesenattrappen (11,11',11"; 21,21',21"; 31,31',31 ") unterschiedliche Größen, insbesondere unterschiedliche Längen aufweisen.

19. Vorrichtung nach den Ansprüchen 4 und 18, **dadurch gekennzeichnet, dass** die Prothesenattrappen (11,11',11"; 21,21',21"; 31,31',31") am Außenumfang des Basisteils (10; 20; 30) jeweils in der radialen Nachbarschaft einer Ausnehmung (12, 12', 12"; 22; 32) zur Aufnahme der Kopfplatte (51) einer Mittelohrprothese angeordnet sind, und dass die Größe der Ausnehmung (12, 12', 12"; 22; 32) der Größe der ihr jeweils benachbarten Prothesenattrappe entspricht.

20. Vorrichtung nach einem der Ansprüche 15 bis 19, **dadurch gekennzeichnet, dass** das Basisteil (10; 20; 30) samt Stegen und Prothesenattrappen (11,11',11"; 21,21',21"; 31,31',31") im Spritzgussverfahren aus Kunststoff gefertigt sind, vorzugsweise einstückig.

## Claims

1. Device for determining the required length of a middle ear prosthesis, having a disc shaped base member (10; 20; 30) to which are attached prosthesis dummies (11,11',11"; 21,21',21"; 31,31',31") of different lengths, which after being detached from the base member (10; 20; 30) can be introduced by means of an applicator into the middle ear of a patient during an operation for the said length to be determined, **characterised in that** accessories are provided on the base member (10; 20; 30) for measurement and/or for performing work on the middle ear prosthesis that is to be implanted, and **in that** the accessories include one or a plurality of recesses (12, 12', 12"; 22; 32) provided in the top face and/or in the bottom face of the disc shaped base member (10; 20; 30) which have an inner diameter that corresponds to the outer diameter of a head plate (51) of the middle ear prosthesis that is to be implanted.

2. Device according to claim 1, **characterised in that**, under every recess (12, 12', 12"; 22; 32), there is provided a cavity (13, 13', 13"; 23; 33) which extends into the base member (10; 20; 30) and which serves especially for receiving a bell (53) that is provided under the head plate (51) of the middle ear prosthesis for attaching the middle ear prosthesis to the stirrup of the middle ear or for receiving a plunger that is provided under the head plate (51) of the middle ear prosthesis to support the middle ear prosthesis on the stirrup foot plate of the middle ear.

3. Device according to one of claims 1 and 2, **characterised in that** the contour of the inner diameter of the
recesses (12, 12', 12"; 22; 32) corresponds at least in part to the outer contour of the head plate (51) of the middle ear prosthesis but in at least one part (14, 14' 14"; 24; 34) is widened radially outwardly as compared to the maximum outer diameter of the head plate (51).

4. Device according to one of claims 1 to 3, **characterised in that** a plurality of recesses (12, 12', 12"; 22; 32) are provided in the base member (10; 20; 30), preferably arranged distributed along the periphery of the base member (10; 20; 30), the said recesses corresponding to the outer diameter of the head plates (51) of middle ear prostheses and are respectively of different depth.

5. Device according to claim 4, **characterised in that** next to every recess (12, 12', 12"; 22; 32) on the base member (10; 20; 30) there is a mark or caption (15, 15', 15"; 25; 35) which correlates with the respective depth of the corresponding recess (12, 12', 12"; 22; 32) or the length of the head plate (51) that can be received in it, and/or with the size of the inner diameter of the corresponding recess (12, 12', 12"; 22; 32) or the outer diameter of the head plate (51), and/or with the shape of the recess (12, 12', 12"; 22; 32) or of the head plate (51).

6. Device according to one of the preceding claims, **characterised in that** the accessories include at least one conical raised part (26; 36) which preferably has a round tip, and which projects away from one face of the base member (20; 30), and which preferably sits in a recess (27), and which is formed for widening the inner diameter of a bell (53) provided under the head plate (51) of the middle ear prosthesis and is formed for attaching the middle ear prosthesis to the stirrup of the middle ear.

7. Device according to claim 6, **characterised in that** the conical raised part (26; 36) is arranged on the same face of the disc shaped base member (20; 30) as the recesses (22; 32) for receiving the head plate (51) of a middle ear prosthesis.

8. Device according to one of the preceding claims, **characterised in that** the accessories include at least one, preferably a plurality, of recesses (42, 42') in the base member (10; 20; 30) which are formed for receiving and if appropriate for measurement and/or performing work on a cartilage or fascia disc that serves as an intermediate insert between the middle ear prosthesis and the ear drum for physical protection of the latter.

9. Device according to claim 8, **characterised in that** the recesses (42, 42') for receiving cartilage or fascia discs have a round or oval contour.

10. Device according to one of claims 8 and 9, **characterised in that** there are provided in the base member (10; 20; 30) a plurality of recesses (42, 42') with inner diameters of different size and/or of different depth.

11. Device according to one of the preceding claims, **characterised in that** the accessories include at least one essentially flat work area (41) on at least one face of the disc shaped base member (10; 20; 30).

12. Device according to claim 11, **characterised in that** a length scale (43) is provided in at least one location of the work area (41) in the surface of the base member (10; 20; 30), preferably calibrated in millimetres.

13. Device according to one of claims 8 to 10 and one of claims 11 and 12, **characterised in that** the recesses (42, 42') for receiving cartilage or fascia discs are provided in the work area (41).

14. Device according to one of claims 1 to 7 and one of claims 8 to 13, **characterised in that** on the one hand the recesses (12, 12', 12"; 22; 32) for receiving the head plate (51) of a middle ear prosthesis; and on the other hand the work area (41) and if appropriate one or a plurality of recesses (42, 42') for receiving cartilage or fascia discs; are arranged on two different, preferably opposite, faces of the disc shaped base member (10; 20; 30).

15. Device according to one of the preceding claims, **characterised in that** the prosthesis dummies (11,11',11"; 21,21',21"; 31,31',31") are attached to the disc shaped base member (10; 20; 30), in the manner of satellites, on arms distributed along its periphery.

16. Device according to claim 15, **characterised in that** protective projections (38), rod shaped especially, are provided distributed along the periphery of the disc shaped base member (30), respectively between two neighbouring prosthesis dummies (31,31',31"), which project beyond the prosthesis dummies (31,31',31") in the radial direction.

17. Device according to claims 15 and 16, **characterised in that** the disc shaped base member (30) has a polygon like, especially hexagonal or octagonal periphery, and **in that** the protective projections (38) project outwards from the corners of the base member (30 in the radial direction).

18. Device according to one of the preceding claims, **characterised in that** the prosthesis dummies (11,11',11"; 21,21',21"; 31,31',31") are of different size, especially of different length.

19. Device according to claims 4 and 18, **characterised in that** the prosthesis dummies (11,11',11"; 21,21',21"; 31,31',31") are arranged at the periphery of the base member (10; 20; 30) respectively radially in the vicinity of a recess (12, 12', 12"; 22; 32) for receiving the head plate (51) of a middle ear prosthesis, and **in that** the size of the recess (12, 12', 12"; 22; 32) corresponds to the size of its respective neighbouring prosthesis dummy.

20. Device according to one of claims 15 to 19, **characterised in that** the base member (10; 20; 30) together with the arms and the prosthesis dummies (11,11',11"; 21,21',21"; 31,31',31") is made of plastics by injection moulding, preferably in one piece.

## Revendications

1. Dispositif pour déterminer la longueur nécessaire d'une prothèse de l'oreille moyenne, comprenant une partie de base (10 ; 20 ; 30) en forme de disque, sur laquelle sont fixées des prothèses factices (11,11',11" ; 21,21',21" ; 31,31',31") de longueur différente, qui peuvent être introduites dans l'oreille moyenne d'un patient au moyen d'un applicateur après le détachement de la partie de base (10 ; 20 ; 30) pour déterminer la longueur pendant une opération, **caractérisé en ce qu'**il est prévu, sur la partie de base (10 ; 20 ; 30), des dispositifs auxiliaires pour mesurer et/ou traiter la prothèse d'oreille moyenne à insérer et **en ce que** les dispositifs auxiliaires comprennent un ou plusieurs évidements (12, 12', 12" ; 22 ; 32) ménagés sur la face supérieure et/ou inférieure de la partie debase (10 ; 20 ; 30) enformededisque, qui présentent un diamètre intérieur correspondant au diamètre externe d'une plaque de tête (51) de la prothèse d'oreille moyenne à insérer.

2. Dispositif selon la revendication 1, **caractérisé en ce qu'**il est prévu, sous chaque évidement (12, 12', 12" ; 22 ; 32), une cavité (13, 13', 13" ; 23 ; 33) dépassant dans la partie de base (10 ; 20 ; 30), qui sert en particulier à recevoir une cloche (53) montée sous la plaque de tête (51) de la prothèse d'oreille moyenne pour fixer la prothèse d'oreille moyenne sur l'étrier de l'oreille moyenne ou recevoir un poinçon monté sous la plaque de tête (51) de la prothèse d'oreille moyenne afin d'appliquer la prothèse d'oreille moyenne sur la plaque de base de l'étrier de l'oreille moyenne.

3. Dispositif selon l'une quelconque des revendications 1 ou 2, **caractérisé en ce que** le contour du diamètre intérieur des évidements (12, 12', 12" ; 22 ; 32) correspond, par segments, au contour externe de la plaque de tête (51) de la prothèse d'oreille moyenne, mais s'étend au moins dans une section (14, 14', 14" ; 24 ; 34) radialement vers l'extérieur par rapport au diamètre externe maximal de la plaque de tête (51).

4. Dispositif selon l'une quelconque des revendications 1 à 3, **caractérisé en ce qu'**il est prévu, dans la partie de base (10 ; 20 ; 30), plusieurs évidements (12, 12', 12" ; 22 ; 32) distribués de préférence sur le pourtour de la partie de base (10 ; 20 ; 30), qui correspondent aux diamètres externes des plaques de tête (51) des prothèses d'oreille moyenne et qui présentent respectivement des profondeurs différentes.

5. Dispositif selon la revendication 4, **caractérisé en ce qu'**il est prévu, sur la partie de base (10 ; 20 ; 30) à côté de chaque évidement (12, 12', 12" ; 22 ; 32), un marquage ou une inscription (15, 15', 15" ; 25 ; 35) qui est en corrélation avec la profondeur respective de l'évidement correspondant (12, 12', 12" ; 22 ; 32) ou avec la longueur de la plaque de tête (51) qui peut y être reçue et/ou avec la grandeur du diamètre intérieur de l'évidement correspondant (12, 12', 12" ; 22 ; 32) ou du diamètre externe de la plaque de tête (51) et/ou avec la forme de l'évidement (12, 12', 12" ; 22 ; 32) ou de la plaque de tête (51).

6. Dispositif selon l'une quelconque des revendications précédentes, **caractérisé en ce que** les dispositifs auxiliaires comprennent au moins une surélévation conique (26 ; 36) avec une pointe de préférence arrondie, qui s'élève d'un côté de la partie de base (20 ; 30), qui se trouve de préférence dans un creux (27 ; 37) et qui est formée pour étendre le diamètre intérieur d'une cloche (53) montée sous la plaque de tête (51) de la prothèse d'oreille moyenne dans le but de fixer la prothèse d'oreille moyenne sur l'étrier de l'oreille moyenne.

7. Dispositif selon la revendication 6, **caractérisé en ce que** la surélévation conique (26 ; 36) est agencée du même côté de la partie de base (20 ; 30) en forme de disque que le ou les évidements (22 ; 32) pour recevoir la plaque de tête (51) d'une prothèse d'oreille moyenne.

8. Dispositif selon l'une quelconque des revendications précédentes, **caractérisé en ce que** les dispositifs auxiliaires comprennent au moins un, de préférence plusieurs,creux (42, 42') dans la partie de base (10 ; 20 ; 30) qui sont formés pour recevoir et éventuellement pour mesurer et/ou traiter une rondelle de cartilage ou de fascia, qui sert de couche intermédiaire entre la prothèse d'oreille moyenne et la membrane tympanique pour la protection mécanique de cette dernière.

9. Dispositif selon la revendication 8, **caractérisé en ce que** les creux (42, 42') destinés à recevoir des rondelles de cartilage ou de fascia présentent un contour rond ou ovale.

10. Dispositif selon l'une quelconque des revendications 8 ou 9, **caractérisé en ce qu'**il est prévu, dans la partie de base (10 ; 20 ; 30), plusieurs creux (42, 42') ayant des diamètres intérieurs de grandeur différente et/ou des profondeurs différentes.

11. Dispositif selon l'une quelconque des revendications précédentes, **caractérisé en ce que** les dispositifs auxiliaires comprennent au moins un champ de préparation (41) sensiblement plan sur au moins une face de la partie de base (10 ; 20 ; 30) en forme de disque.

12. Dispositif selon la revendication 11, **caractérisé en ce qu'**on applique, au moins en un point du champ de préparation (41), une échelle de longueur (43) à la surface de la partie de base (10 ; 20 ; 30), qui est étalonnée de préférence en millimètres.

13. Dispositif selon l'une quelconque des revendications 8 à 10 et selon l'une quelconque des revendications 11 ou 12, **caractérisé en ce que** les creux (42, 42') sont ménagés pour recevoir des rondelles de cartilage ou de fascia dans le champ de préparation (41).

14. Dispositif selon l'une quelconque des revendications 1 à 7 et selon l'une quelconque des revendications 8 à 13, **caractérisé en ce que** les évidements (12, 12', 12" ; 22 ; 32) sont ménagés pour recevoir la plaque de tête (51) d'une prothèse d'oreille moyenne, d'une part, et le champ de préparation (41), ainsi qu'éventuellement un ou plusieurs creux (42, 42') pour recevoir des rondelles de cartilage ou de fascia, d'autre part, sur deux côtés différents, de préférence opposés, de la partie de base (10 ; 20 ; 30) en forme de disque.

15. Dispositif selon l'une quelconque des revendications précédentes, **caractérisé en ce que** les prothèses factices (11,11',11" ; 21,21',21" ; 31,31',31") sont fixées distribuées en satellites sur des barrettes sur le pourtour externe de la partie de base (10 ; 20 ; 30) en forme de disque.

16. Dispositif selon la revendication 15, **caractérisé en ce qu'**il est prévu, sur le pourtour externe de la partie de base (30) en forme de disque, de manière distribuée respectivemententredeuxprothèses factices (31, 31', 31") adjacentes, des saillies de protection (38) en particulier en forme de tige qui dépassent des prothèses factices (31, 31', 31") dans la direction radiale.

17. Dispositif selon les revendications 15 et 16, **caractérisé en ce que** la partie de base (30) en forme de disque présente un pourtour externe polygonal, en particulier hexagonal ou octogonal, et **en ce que** les saillies de protection (38) s'élèvent dans la direction radiale depuis les coins de la partie de base (30) vers l'extérieur.

18. Dispositif selon l'une quelconque des revendications précédentes, **caractérisé en ce que** les prothèses factices (11,11',11" ; 21,21',21" ; 31,31',31") présentent des tailles différentes, en particulier des longueurs différentes.

19. Dispositif selon les revendications 4 et 18, **caractérisé en ce que** les prothèses factices (11,11', 11" ; 21,21',21" ; 31,31',31") sont agencées sur le pourtour externe de la partie de base (10 ; 20 ; 30), respectivement, à proximité radiale d'un évidement (12, 12', 12" ; 22 ; 32) pour recevoir la plaque de tête (51) d'une prothèse d'oreille moyenne et **en ce que** la taille de l'évidement (12, 12', 12" ; 22 ; 32) correspond à la taille de sa prothèse factice respectivement adjacente.

20. Dispositif selon l'une quelconque des revendications 15 à 19, **caractérisé en ce que** la partie de base (10 ; 20 ; 30) avec les barrettes et les prothèses factices (11,11',11" ; 21,21',21" ; 31, 31', 31") est fabriquée selon le procédé de moulage par injection à partir d'un matériau synthétique, de préférence d'un seul tenant.
